Europäisches Patentamt

European Patent Office

Office européen des brevets

(11) Veröffentlichungsnummer: **0 504 754 A1**

## (12) EUROPÄISCHE PATENTANMELDUNG

(21) Anmeldenummer: **92104359.2**

(22) Anmeldetag: **13.03.92**

(51) Int. Cl.5: **A61K 7/043**

(30) Priorität: **16.03.91 DE 4108664**

(43) Veröffentlichungstag der Anmeldung:
**23.09.92 Patentblatt 92/39**

(84) Benannte Vertragsstaaten:
**BE DE ES FR GB IT LU NL**

(71) Anmelder: **DR. SCHELLER COSMETICS GmbH
Schillerstrasse 21-27
W-7332 Eislingen(DE)**

(72) Erfinder: **Häring, Lothar
Haldenstrasse 19
W-7457 Wessingen(DE)**

(74) Vertreter: **Werner, Hans-Karsten, Dr. et al
Deichmannhaus am Hauptbahnhof
W-5000 Köln 1(DE)**

(54) **Nagellack und Verwendung desselben.**

(57) Es wird ein Nagellack beschrieben enthaltend wäßrige, filmbildende Kunststoffdispersionen, sowie gegebenenfalls Füllstoffe, Verdickungsmittel, Pigmente, Farbstoffe, pH-Regulatoren, Entschäumer, Netzmittel und sonstige übliche Hilfsmittel, wobei er mindestens 0,5 Gew.-%, vorzugsweise 1 bis 10 Gew.-% einer wasserlöslichen und/oder mit Wasser mischbaren und dabei wasserspeichernden Substanz enthält.

EP 0 504 754 A1

Gegenstand der vorliegenden Erfindung ist ein Nagellack enthaltend wäßrige, filmbildende Kunststoffdispersionen sowie gegebenenfalls Füllstoffe, Verdickungsmittel, Pigmente, Farbstoffe, pH-Regulatoren, Entschäumer, Netzmittel und sonstige übliche Hilfsmittel. Weiterhin betrifft die Erfindung die Verwendung dieses Nagellacks als Grundlack, auf dem anschließend ein anderer Nagellack aufgetragen werden kann.

Es ist bekannt, daß die üblichen Nagellacke, welche mit Hilfe organischer Lösungsmittel gelöst auf die Fingernägel aufgebracht werden, prinzipiell auch durch wäßrige Kunststoffdispersionen ersetzt werden können, wobei eine Lackschicht entsteht, die nicht mehr mit organischen Lösungsmitteln als Nagellackentferner entfernt werden muß, sondern als Film abgezogen werden kann. Derartige Nagellacke sind beispielsweise beschrieben in der EP-A-0 143 480, der EP-A-0 391 322 sowie der DE-A-39 31 237. Ein weiteres Nagelüberzugsmittel ist in der DE-A-27 57 773 beschrieben. Die US-A-4 666 709 beschreibt ein Nagelüberzugsmittel niedriger Viskosität, welches mit einem Filzstift aufgebracht werden kann. Es besteht aus einem alkalilöslichen Polyester mit hoher Säurezahl und einem wäßrig-alkalischen Lösungsmittel. Es kann nur mit Alkali oder organischen Lösungsmitteln entfernt werden.

Ein wesentlicher Nachteil aller dieser abziehbaren und mit Wasser verdünnbaren Nagellacke besteht darin, daß sie sich inbesondere an der Nagelspitze vorzeitig ablösen, da diese den stärksten mechanischen Belastungen unterworfen ist. Die bisher auf den Markt gebrachten abziehbaren Nagellacke auf Basis wäßriger Kunststoffdispersionen haben daher die Verbraucher nicht wirklich zufriedengestellt.

Sofern die Rezepturen so eingestellt wurden, daß sie stärker am Fingernagel haften, führte dies dazu, daß sie nach längerem Aushärten überhaupt nicht mehr vom Fingernagel entfernbar waren, und zwar weder als Film abziehbar noch mit organischen Lösungsmitteln.

Die Erfindung hat sich somit die Aufgabe gestellt, einen Nagellack auf Basis wäßriger filmbildender Kunststoffdispersionen zur Verfügung zu stellen, welcher ausreichend gut haftet, und zwar insbesondere an den Nagelspitzen, und dennoch später problemlos als Film abgezogen werden kann, insbesondere wenn er zusätzlich mit einem anderen Nagellack überstrichen worden ist. Die Erfindung hat sich somit insbesondere die Aufgabe gestellt, einen Nagellack zu entwickeln, der als Grundlack eingesetzt werden kann und auf den anschließend jeder andere Nagellack aufgetragen werden kann. Dabei soll dann auch diese doppelte Schicht ohne organische Lösungsmittel entfernbar sein und als Film abgezogen werden können. Schließlich soll dieser Grundlack den Fingernagel pflegende Substanzen wie Vitamine, Proteine, Harnstoff, UV-Schutzmittel enthalten können. Weiterhin soll er nagelhärtende Substanzen wie Formaldehyd oder antimykotische Substanzen enthalten können.

Diese Aufgabe kann überraschend dadurch gelöst werden, daß mindestens 0,5 Gew.-%, vorzugsweise 1 bis 10 Gew.-% einer wasserlöslichen und/oder mit Wasser mischbaren und dabei wasserspeichernden Substanz zugesetzt werden. Bewährt haben sich insbesondere Glycerin sowie gegebenenfalls substituierte und/oder hydrierte Mono-, Di- und Oligosaccharide sowie Gemische derselben. Es war zunächst schon nicht vorhersehbar, daß es möglich ist, trotz dieser Zusätze brauchbare Nagellacke zu entwickeln, da man sich bisher stets bemüht hat, die Wasserempfindlichkeit der fertigen Nagellackschicht so gering wie möglich zu halten. Man hat daher nach Möglichkeit den Zusatz von Emulgatoren und anderen Substanzen vermieden, welche den fertigen Nagelack durch Wasser lösbar oder quellbar machen. Überraschenderweise wird aber durch den erfindungsgemäßen Zusatz der wasserlöslichen und/oder mit Wasser mischbaren und dabei wasserspeichernden Substanzen ein völlig unerwarteter Effekt erzielt. Die erfindungsgemäßen Nagellacke haften nämlich an den vorderen Nagelspitzen wesentlich stärker als auf der übrigen Fläche, so daß sie einerseits als Film abziehbar sind, andererseits an der Fingernagelspitze wesentlich stärker haften. Dies gilt insbesondere dann, wenn der erfindungsgemäße Nagellack als Grundlack verwendet wird, auf den ein anderer Nagellack aufgetragen wurde. Versuche mit den bisher bekannten Nagellacken auf Basis von wässrigen Kunststoffdispersionen als Grundlack haben hingegen gezeigt, daß diese nach einer Trocknungszeit von 4 bis 8 Stunden nicht mehr abgezogen werden können und somit nur noch mechanisch entfernbar sind.

Obwohl diese Eigenschaften noch immer nicht ganz erklärbar sind, wird inzwischen angenommen, daß diese Effekte darauf beruhen, daß die Teile des Fingernagels, die mit dem Nagelbett verbunden sind, atmen und Feuchtigkeit transpirieren, während die Fingernagelspitzen austrocknen und praktisch keine Feuchtigkeit mehr abgeben. Genauere Untersuchungen haben ergeben, daß die erfindungsgemäßen Nagellacke nicht als Dampfsperre wirken. Die mit dem erfindungsgemäßen Nagellack beschichteten Fingernägel können somit weiterhin Feuchtigkeit abgeben und transpirieren. Diese Feuchtigkeit ist offensichtlich in der Lage, zusammen mit den erfindungsgemäß zugesetzten wasserlöslichen und/oder mit Wasser mischbaren und dabei wasserspeichernden Substanzen als Weichmacher für den Nagellack zu wirken, so daß die Nagellackschicht auch nach längerer Zeit und bei zusätzlicher Beschichtung mit anderem Nagellack als Filmhäutchen abgezogen werden kann. Im Bereich der nicht mehr mit dem Nagelbett verbundenen Fingernagelspitze kann der Nagellack stärker austrocknen, so daß er dort stärker haftet. Diese stärkere Haftung bewirkt eine höhere Strapazierfähigkeit an den besonders strapazierten Stellen der Nagellackschicht.

Diese Eigenschaften des erfindungsgemäßen Nagellacks machen ihn besonders geeignet, als Grundlack verwendet zu werden. Da eine getrocknete Schicht des erfindungsgemäßen Nagellacks unempfindlich ist gegen organische Lösungsmittel, können nämlich auf diesem Grundlack auch herkömmliche Nagellacke aufgetragen werden. Diese Nagellacke enthalten zwar noch organische Lösungsmittel, können jedoch ohne Anwendung von organischen Lösungsmitteln wieder entfernt werden, da sie zusammen mit dem weichbleibenden Grundlack als Film abgezogen werden können. Es ist daher möglich, mit dem erfindungsgemäßen Nagellack großflächig die gesamte Nagelfläche abzudecken, wobei man dann vor allem hautfarbene oder wenig gefärbte Rezepturen anwendet. Auf dieser Schicht des Grundlacks kann dann mit stärker gefärbten Nagellacken dekorativ das Erscheinungsbild der Fingernägel variiert werden.

Als wäßrige filmbildende Kunststoffdispersionen können prinzipiell alle schon jetzt für kosmetische Zwecke beschriebenen Kunststoffdispersionen verwendet werden. Besonders bewährt haben sich Kunststoffdispersionen aus Polyvinylacetat und Polyurethan. Als Füllstoff hat sich insbesondere Talkum bewährt. Als Verdickungsmittel geeignet sind vor allem nicht filmbildende Kunststoffdispersionen sowie hochdisperse Kieselsäuren. Als Pigmente werden die üblichen Pigmente verwendet, wobei mit Metalloxid beschichtete Glimmer als sogenannte Perlglanzpigmente besonders bevorzugt sind. Als Farbstoffe kommen alle üblichen Farbstoffe für Nagellacke in Frage. Zur Herstellung eines Grundlacks empfehlen sich insbesondere hautfarbige Tönungen, auf die dann gewünschtenfalls kräftiger gefärbte Nagellacke zur Dekoration aufgetragen werden können. Als pH-Regulatoren kommen insbesondere Ammoniak, Amine oder schwache Laugen in Frage, da die Kunststoffdispersionen von der Herstellung her oft einen zu niedrigen pH-Wert aufweisen. Weiterhin können üblicherweise Entschäumer, beispielsweise auf Basis höherer Fettsäuren eingesetzt werden. Auch übliche Hilfsmittel und Netzmittel können zum Einsatz kommen. Erfindungswesentlich ist allein, daß durch den Zusatz der wasserlöslichen oder mit Wasser mischbaren und dabei wasserspeichernden Substanzen zusammen mit der vom lebenden Fingernagel abgegebenen Feuchtigkeit ein Weichmachereffekt erzielt wird, welcher die Haftfestigkeit am Fingernagel etwas herabsetzt und dadurch das Abziehen als Film ermöglicht.

Die erfindungsgemäßen Nagellacke können so hergestellt werden, daß sie keine oder nahezu keine organischen Lösungsmittel sowie keine Emulgatoren enthalten. Dies beeinflußt in erheblichem Maße die Trocknungseigenschaften und die Aushärtungseigenschaften sowie die Empfindlichkeit der fertigen Nagellackschicht gegen Wasser beim Waschen der Hände etc.. Ein besonderer Vorteil der erfindungsgemäßen Nagellacke besteht darin, daß in den Lack auch die Fingernägel pflegende Zusätze eingearbeitet werden können. Geeignet sind beispielsweise Vitamine wie Panthenol, Proteine, Harnstoff, UV-Schutzmittel und härtende Zusätze wie Aldehyde und Hydrolysate aus Chitin und/oder Keratin. Auch antimykotische Zusätze sind möglich.

Versuche mit einem erfindungsgemäßen Nagellack unter Zusatz von Fluorescein und anschließende Untersuchung der durch einfaches Abziehen des Filmes wieder freigelegten Fingernägel haben ergeben, daß derartige wasserlösliche Substanzen in die Nagelsubstanz eindringen, ohne klinisch-dermatologisch pathologische Veränderungen an der Nageloberfläche zu hinterlassen. Die Bestandteile des Nagellacks dringen sowohl in den klinisch gesunden als auch in den besonders trockenen, spröden, d.h. Onychorrhexis-Nagel ein. Durch die Markierung mit Fluorescein konnte nachgewiesen werden, daß derartige Bestandteile des Nagellacks extrem lange innerhalb der Nagelsubstanz verweilen. Auch 3 Wochen nach der zweiten vierstündigen Applikation waren noch sehr deutliche Einlagerungen innerhalb der Nagelsubstanz vorhanden. Die Intensität der Fluoreszenz betrug nach 3 Wochen noch ca. 50 % der Anfangsintensität unmittelbar nach Entfernung der zweiten Applikation. Die Fluoreszenz war in den Nägeln stets flächig nachweisbar. Dies beweist, daß der Nagellack ein flächiges Eindringen seiner Bestandteile in die Nagelsubstanz ermöglicht.

Diese Befunde sind von großer kosmetischer und medizinischer Bedeutung, da sie beweisen, daß es möglich ist, mittels des Nagellacksals Träger- oder Grundlack Wirkstoffe aus dem kosmetischen und aus dem medizinischen Bereich in die Nagelsubstanz einzubringen. Dies ermöglicht nicht nur die ursächliche Korrektur kosmetisch störender Nagelveränderungen, sondern auch die gezielte Therapie medizinisch definierter Nagelveränderungen.

In den nachfolgenden Beispielen sind einige erfindungsgemäße Rezepturen zusammengestellt, die sich bereits gut bewährt haben. Darüber hinaus wurde festgestellt, daß auch bei anderen Rezepturen mit dem erfindungsgemäßen Zusatz der wasserlöslichen und/oder mit Wasser mischbaren und dabei wasserspeichernden Substanzen eine unterschiedliche Haftfestigkeit des Nagellacks erzielt wird, einerseits im Gesamtbereich und andererseits an den Fingernagelspitzen, die nicht mehr mit dem Nagelbett in Verbindung stehen.

Beispiel 1

| Polyvinylacetat-Dispersion, feinstdispers, mit einem Feststoffgehalt von 55 Gew.-% | 75.000 Gew.-% |
|---|---|
| 50%-ige wäßrige Saccharose-Lösung | 6.400 Gew.-% |
| pH-Regulator (Ammoniak oder untoxisches Amin) | 0.300 Gew.-% |
| Entschäumer (Fettsäurederivat) | 0.300 Gew.-% |
| Hochdisperse Kieselsäure | 0.600 Gew.-% |
| Talkum | 8.000 Gew.-% |
| Wasser | 9.400 Gew.-% |

Es handelt sich um einen Nagellack mit natürlicher Mattigkeit, der je nach Schichtdicke innerhalb von 2 bis 10 Minuten trocknet und danach bereits als dünner Film abgezogen werden kann. Diese Nagellackschicht kann auch nach Tagen noch als Film abgezogen werden, haftet jedoch an den Fingernagelspitzen stärker und kann dort gegebenenfalls mechanisch vollständig entfernt werden.

Beispiel 2

| Wäßrige Polyurethandispersion (aliphatisch) mit einem Feststoffgehalt von 40% | 80.000 Gew.-% |
|---|---|
| N-2-Methylpyrrolidon | 4.000 Gew.-% |
| Glycerin (99%-ig) | 3.800 Gew.-% |
| Polyurethanverdicker mit einem Feststoffgehalt von 10% in Wasser (Bochigel L 75) | 3.000 Gew.-% |
| Hochdisperse Kieselsäure | 0.600 Gew.-% |
| Entschäumer | 0.300 Gew.-% |
| Talkum | 8.300 Gew.-% |

Es handelt sich wiederum um einen zunächst farblosen Grundlack mit natürlicher Mattigkeit. Er kann mit den verschiedensten üblichen Farbstoffen abgetönt oder stark angefärbt werden. Als Nagellack aufgetragen, trocknet er innerhalb von 2 bis 10 Minuten und kann danach als dünne Filmhaut abgezogen werden. Er haftet an den Fingernagelspitzen stärker als auf der übrigen Fläche des Fingernagels.

Zu der Rezeptur des Beispiels 2 wurden 6 Gew.-% einer 50%-igen wäßrigen Methacrylatdispersion zugegeben. Dies führte zu einer verbesserten Haftfähigkeit, ohne die Abziehbarkeit des Films zu beeinträchtigen. Auch diese Rezepturen können beliebig eingefärbt und dann auch unmittelbar als Dekorlack eingesetzt werden. Als Grundlack eingesetzt, bieten sie einen guten Schutz gegen die organischen Lösungsmittel von nachträglich aufgetragenen üblichen Nagellacken. Auch diese nachträglich aufgetragenen Nagellackschichten können zusammen mit dem Grundlack als dünnes Filmhäutchen abgezogen werden. Der Grundlack bietet somit einen guten Schutz gegen die organischen Lösungsmittel, insbesondere bei hiergegen empfindlichen Anwenderinnen. Auf Nagellackentferner kann vollständig verzichtet werden.

Beispiel 3

| Wäßrige Polyurethandispersion (aliphatisch) mit einem Feststoffgehalt von 40% | 79.800 Gew.-% |
|---|---|
| N-2-Methylpyrrolidon | 4.000 Gew.-% |
| Glycerin (99%-ig) | 2.000 Gew.-% |
| Sorbit (70 % in Wasser) | 2.000 Gew.-% |
| Polyurethanverdicker mit einem Feststoffgehalt von 10% in Wasser (Bochigel L 75) | 3.000 Gew.-% |
| Hochdisperse Kieselsäure | 0.600 Gew.-% |
| Entschäumer | 0.300 Gew.-% |
| Talkum | 8.300 Gew.-% |

Beispiel 4

| Wäßrige Styrol-acrylat-Dispersion (60 %) | 6.300 Gew.-% |
| Wäßrige Polyurethandispersion (aliphatisch) mit einem Feststoffgehalt von 40% | 82.780 Gew.-% |
| Sorbit (70 % in Wasser) | 2.750 Gew.-% |
| Entschäumer (Fettsäurederivat) | 0.250 Gew.-% |
| Hochdisperse Kieselsäure | 0.500 Gew.-% |
| Talkum, weiß DAB | 7.400 Gew.-% |
| Netzmittel | 0.020 Gew.-% |

Zu 99,4 Teilen dieser Grundlage wurden je 0,2 Teile Ca-D-Pantothenat, Keratin-Hydrolysat und Chitin-Hydrolysat zugemischt.

Der Nagellack gemäß Beispiel 3 wurde in einem Verbrauchertest mit mehreren hundert Verbraucherinnen untersucht und führte zu folgenden Ergebnis:

Gesamtbeurteilung des Nagellacksystems:

30 % entfielen auf "sehr gut"
50 % entfielen auf "gut"

Haltbarkeit nach 48 Stunden:

19 % entfielen auf "sehr gut - Lack ist perfekt"
47 % entfielen auf "gut - Lack ist wenig abgenutzt"

Bewertung des Peel-off-Effektes:

36 % entfielen auf "Lack läßt sich an einem Stück abziehen"
40 % entfielen auf "Lack läßt sich teilweise abziehen,
Spitzen mußten leicht abgekratzt werden"

**Patentansprüche**

1. Nagellack enthaltend eine wäßrige filmbildende Kunststoffdispersion sowie gegebenenfalls Füllstoffe, Verdickungsmittel, Pigmente, Farbstoffe, pH-Regulatoren, Entschäumer, Netzmittel und sonstige übliche Hilfsmittel, **dadurch gekennzeichnet**, daß er mindestens 0,5 Gew.-%, vorzugsweise 1 bis 10 Gew.-% einer wasserlöslichen und/oder mit Wasser mischbaren und dabei wasserspeichernden Substanz enthält.

2. Nagellack gemäß Anspruch 1, **dadurch gekennzeichnet**, daß die Substanz Glyzerin ist oder ein gegebenenfalls substituiertes und/oder hydriertes Mono-, Di-, Oligosaccharid oder Gemische derselben.

3. Nagellack gemäß Anspruch 1 oder 2, **dadurch gekennzeichnet**, daß die filmbildende Kunststoffdispersion ein Polyvinylacetat oder ein Polyurethan ist.

4. Nagellack gemäß einem der Ansprüche 1 bis 3, **dadurch gekennzeichnet**, daß er keine oder nahezu keine organischen Lösungsmittel und/oder Emulgatoren enthält.

5. Nagellack gemäß einem der Ansprüche 1 bis 4, **dadurch gekennzeichnet**, daß er zusätzlich den Fingernagel pflegende, härtende oder schützende Zusätze enthält.

6. Verwendung von Nagellacken gemäß einem der Ansprüche 1 bis 5 als Grundlack, auf dem anschließend ein anderer Nagellack aufgetragen werden kann.

Europäisches
Patentamt

**EUROPÄISCHER RECHERCHENBERICHT**

Nummer der Anmeldung

EP   92 10 4359

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 126 144 (P. A. DUARTE) <br> * Spalte 1 * <br> * Spalte 2, Zeile 1 - Zeile 30 * <br> * Spalte 2, Zeile 58 - Zeile 68 * <br> * Spalte 3, Zeile 1 - Zeile 36 * <br> * Spalte 4, Zeile 63 - Zeile 65 * <br> * Spalte 5, Zeile 1 - Zeile 4 * <br> * Ansprüche 1,4,5,6,9,10-22 * <br> --- | 1-3,5-6 | A61K7/043 |
| X | PATENT ABSTRACTS OF JAPAN <br> vol. 10, no. 107 (C-341)(2164) 22. April 1986 <br> & JP-A-60 237 010 ( SHISEIDO K.K. ) 25. November 1985 <br> * Zusammenfassung * <br> --- | 1,5,6 | |
| A | EP-A-0 291 555 (SHISEIDO COMPANY LIMITED) <br> * Seite 1 - Seite 6, Zeile 31 * <br> * Ansprüche 1,7,8,9 * <br><br> ----- | 1-6 | |
| | | | RECHERCHIERTE SACHGEBIETE (Int. Cl.5) <br><br> A61K |

Der vorliegende Recherchenbericht wurde für alle Patentansprüche erstellt

| Recherchenort | Abschlußdatum der Recherche | Prüfer |
|---|---|---|
| DEN HAAG | 06 MAI 1992 | SIERRA GONZALEZ |